# EUROPEAN PATENT SPECIFICATION

(11) **EP 1 427 465 B1**
(45) Date of publication and mention of the grant of the patent: **13.01.2016**
(21) Application number: 02764384.0
(22) Date of filing: 18.09.2002
(51) Int. Cl.: A61M 15/00

(54) **POWDER INHALER**
INHALATEUR DE POUDRE
PULVERINHALATOR

(30) Priority: 19.09.2001 AU PR783001; 19.09.2001 AU PR776601; 19.09.2001 AU PR776701; 13.06.2002 AU PR293802
(43) Date of publication of application: 16.06.2004
(73) Proprietor: Advent Pharmaceuticals Pty Limited, Notting Hill, VIC 3168 (AU)
(72) Inventor: ALLAN, Robert, David, Greensborough, VIC 3088 (AU); PIKE, Gregory, Charles, Dandenong, VIC 3175 (AU)
(74) Representative: Makovski, Priscilla Mary
(86) International application number: PCT/AU2002/001284
(87) International publication number: WO 2003/024514

(56) References cited:
- WO-A1-01/28616
- WO-A2-02/24263
- WO-A2-02/24263
- FR-A1- 2 662 936
- GB-A- 2 264 237
- US-A- 6 065 472
- US-B1- 6 273 085

## Description

### INTRODUCTION

This invention relates to inhalers and in particular to inhalers for use with powered medicaments.

### BACKGROUND OF THE INVENTION

There are many types of inhalers that can provide delivered metered doses. The majority of inhalers of this kind are designed to provide multiple doses. It is however known that inhalers of this kind can also be used to provide a single dose.

In situations where a metered dose is to be dispensed it is important that the inhaler always dispenses the exact dose. There is also a problem with inhalers of this kind if there is a tendency to allow unintentional additional dosing. Inhalers need to be small, compact, easy to use and yet not too expensive. The inhalers also need to satisfy safety criteria set down by appropriate standards.

It is these issues that have brought about the present invention.

One known device is shown in WO 02/24263, which discloses an inhalation device for delivering unit doses of powdered medicament having a removably mounted cartridge with a plurality of compartments each for containing a discrete unit dose and a sealing layer for closing the compartments, and a separator for removing the sealing layer from a respective compartment to open an air flow passageway defined by the compartment and the separator. The document WO 02/24263 is state of the art according to Art. 54(3) EPC.

The documents GB 2 264 237, FR 2 662 936 and US 6 065 472 disclose other inhalation devices for delivering unit doses of powdered medicament.

### SUMMARY OF THE INVENTION

In accordance with the present invention there is provided an inhaler for delivering metered doses of powdered medicament, the inhaler having a plurality of compartments spaced in an annular array and each containing a metered dose of the medicament, a lever to displace the compartments one by one into line with an inhalation aperture that constitutes a mouthpiece, each compartment including radially inner and outer edges, the plurality of compartments being closed by a sealing layer, characterised in that the inhaler has a mechanism adapted to lift the inner and outer edges of the sealing layer off the radially inner and outer edges of each compartment one by one as it is indexed to align with the inhalation aperture to open an air passageway defined by the compartment and the sealing layer so that, in use, on inhalation through the mouthpiece, air flow in the air flow passageway picks up and entrains the powder in the compartment to be drawn with the air out of the inhaler through the mouthpiece.

Further features of the invention are specified in the subsidiary claims.

### DESCRIPTION OF THE DRAWINGS

Embodiments of the present invention will now be described by way of example only in which:
Figure 1 is an exploded view perspective view of an inhaler in accordance with a first embodiment of the invention,
Figure 2A & 2B are perspective views of the inhaler,
Figure 3 is an exploded perspective view of the inhaler that shows a lid of the inhaler in an open position with a cartridge outside the inhaler,
Figure 4 is an exploded perspective view of the cartridge,
Figure 5 is a perspective view of a lower cover of the cartridge,
Figure 6 is a perspective view of the underside of a base of the cartridge,
Figure 7 is a perspective view of the underside of a upper cover of the cartridge,

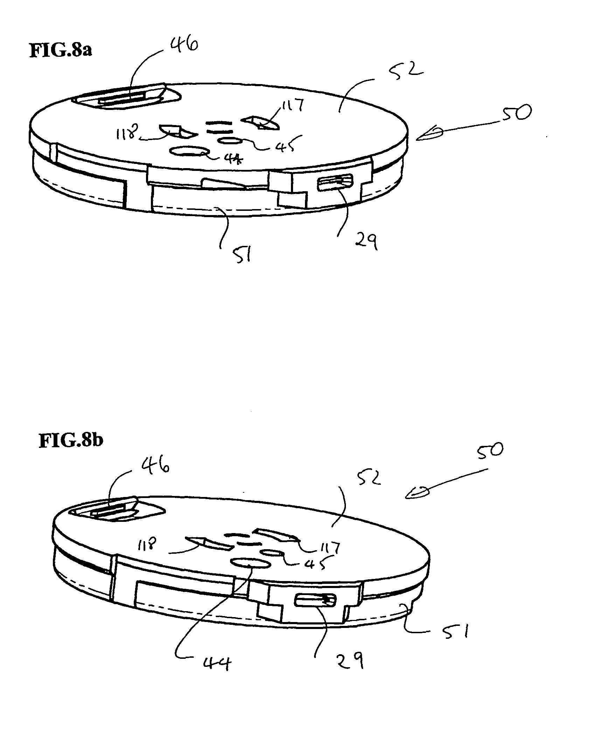

Figures 8a and 8b are perspective views of the assembled cartridge viewed from the top,
Figure 9 is a plan view of the cartridge,
Figure 10 is a cross sectional view taken along the lines A-A of Figure 9,
Figure 11 is a perspective view of the cartridge with part cutaway showing an open compartment,
Figure 12 is a perspective view with part cut away of the assembled inhaler showing the air passageway,
Figures 13a and 13b are perspective views of the top of the inhaler with part of a cover cut away, and

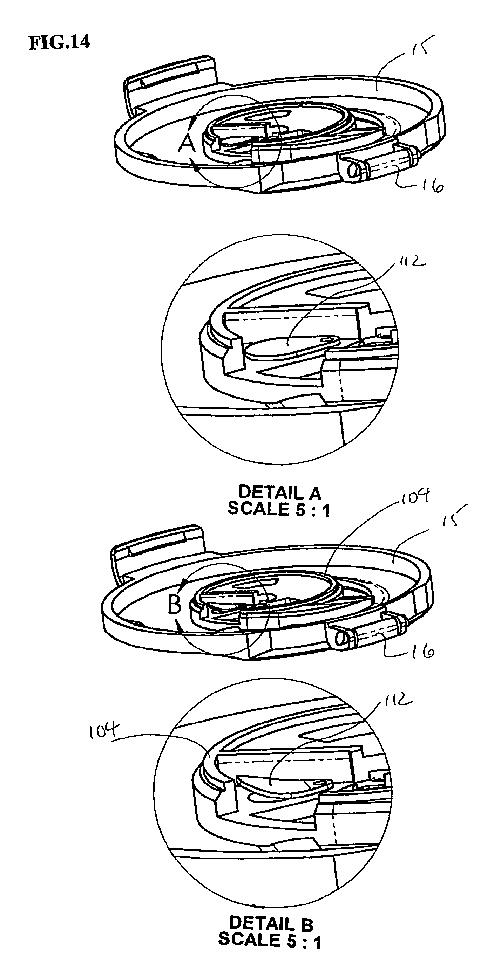

Figures 14 are perspective views of the underside of the lid of the inhaler,
Figure 15 is an exploded perspective view of a cartridge for an inhaler in accordance with a second embodiment of the invention,
Figure 16 is a perspective view of an upper cover of the cartridge of Figure 15,
Figure 17 is a cross sectional view taken along the lines 8-8 of Figure 16,
Figure 18 is an exploded perspective view of a cartridge for an inhaler in accordance with a third embodiment of the invention, and
Figure 19 is an enlarged perspective view of the mouth of the cartridge shown in Figure 18.

### DESCRIPTION OF THE PREFERRED EMBODIMENTS

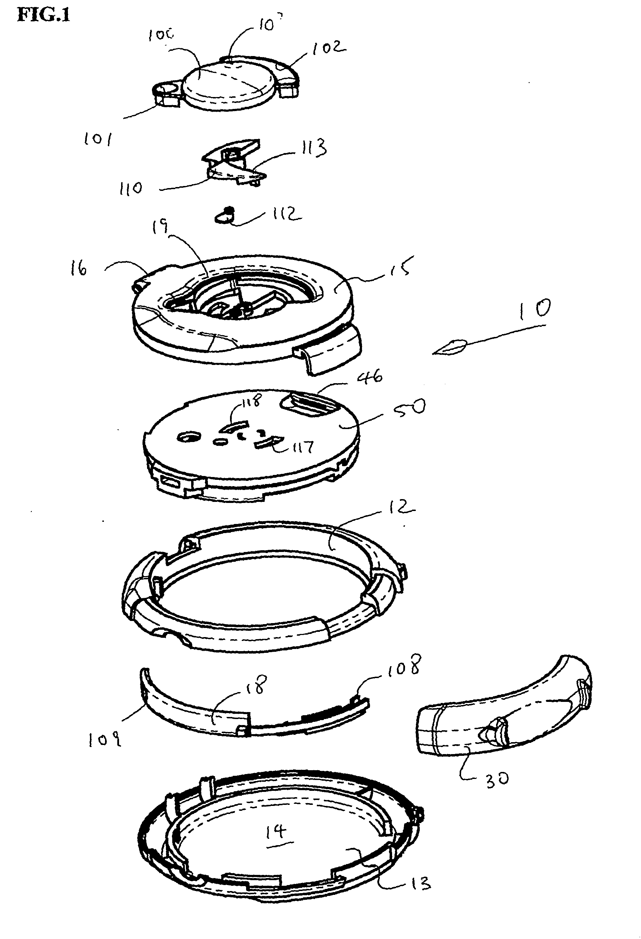

As shown in the exploded view of Figure 1 an inhaler 10 comprises a disposable medicament cartridge 50 that is located in an inhaler body 11 that includes an upper body 12, lower body 13 that fit together to support a drive cam 18 and lever 30 and define a recess 14 into which the cartridge 50 fits. The body 11 is closed by a lid 15 that is hinged to one side 16 of the body 11. The upper surface of the lid supports a window 100, air entry inlet 19 and air entry indicator 110 and a one way valve 112.

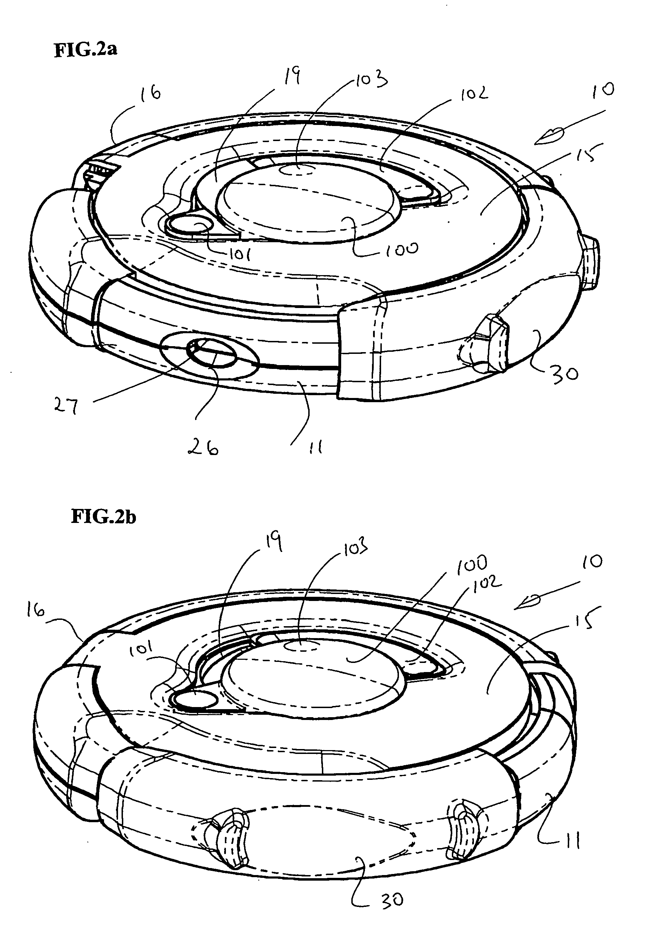

As shown in Figure 2, the inhaler 10 is substantially circular in plan and has a mouthpiece 26 having an inhalation aperture 27 positioned in the body periphery on one side with the displacement lever 30 located on that side to be displaceable relative to the body 11 between the open position shown in Figure 2a to the closed position in which the lever 30 covers the mouthpiece 26 as shown in Figure 2b.

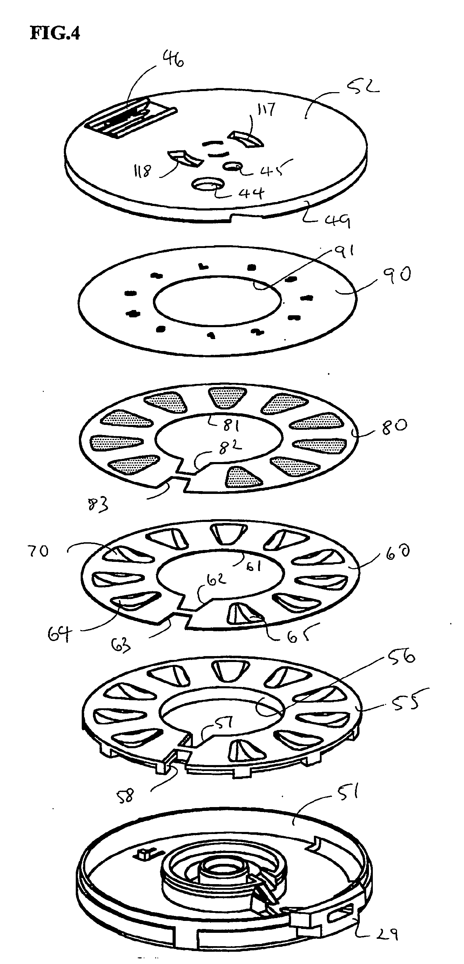

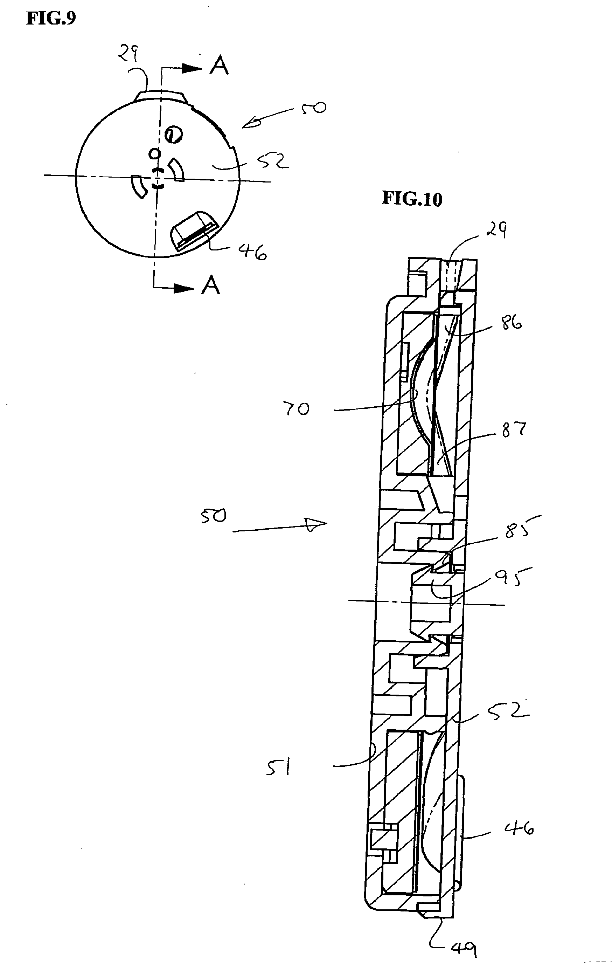

The cartridge 50 is shown in greater detail in Figure 4 and comprises a multi-layered annular disc assembly that is located between upper and lower covers 52, 51 that clip together as shown in Figure 10. The upper cover 52 has an air inlet aperture 45 that communicates with the disc assembly to define an air passageway that exits the cartridge via slot 29 on the periphery of the lower cover 51. The disc assembly includes a cartridge base 55 that is of disc shape with a central aperture 56. The cartridge base 55 supports a base foil 60 that has a central aperture 61 and contains ten recessed compartments 70 spaced around the periphery of the base foil 60. The compartments are equally spaced except that there is a wider gap between the first 64 and the last 65 compartments. The base 55 is formed with recesses that correspond to the compartments 70.

The base foil 60 is covered first by a perforated layer 80 which is in turn covered by a lidding foil layer 90. Both the perforated layer and lidding foil 80 and 90 have internal apertures 81 and 91. The apertures 56, 61, 81 in the cartridge base 55, base foil 60 and perforated layer 80 include a cut-out 57, 62 and 82 that is radically aligned with a cut-out 58, 63, and 83 in the outer periphery. The lidding foil 90 is securely bonded to the perforated layer 80 which is attached to the base foil 60 to seal the compartments 70 once filled with medicament powder.

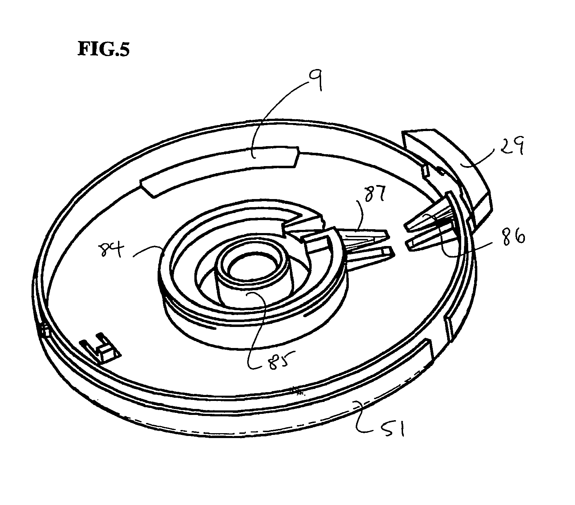

The cartridge 50 is designed to hold a plurality of metered doses of powdered medicament in separate sealed compartments 70 and the operation of the lever 30 displaces the drive cam 18 which rotates components of the cartridge 50 to expose individual doses to the air passageway that is in communication with the mouthpiece 26. As shown in Figure 5, the cartridge 50 includes openers 86, 87 located in the lower cover 51 that operates to expose a single dose by unsealing each compartment 70 so that when the user inhales through the mouthpiece 26 air is drawn through an aperture in the lid 15 of the inhaler 10 through the inlet aperture of the cartridge, across the unsealed compartment 70 to pick up the powder in the compartment 70 for delivery to the mouthpiece 26 via the outlet slot 29. The cartridge 50 can be disposed of and be replaced by a new cartridge when all or part of the compartments 70 of the powered medicament have been emptied. The inhaler is in consequence reusable. The cartridge may be removed or reinserted as required.

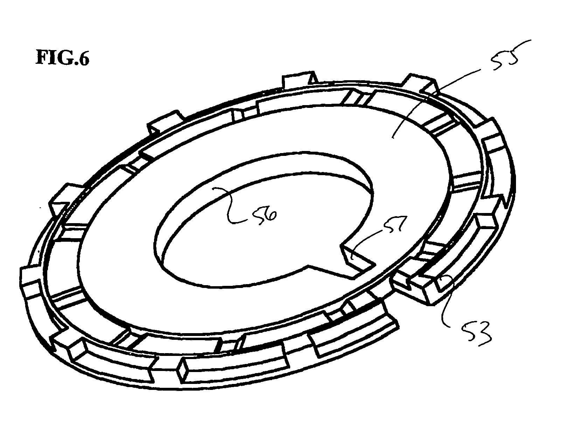

As shown in Figure 6, the underside of the periphery of the cartridge base 55 is provided with a plurality of equally spaced cutouts 53, that are adapted to be engaged by the drive cam 18 driven by the lever 30 to cause the cartridge base 55, base foil 60, perforated layer 80, lidding foil 90 and upper cover 52 to be rotated through a small angle when the lever 30 is displaced in the anti-clockwise direction. The upper cover 52 is also rotated by the drive cam 18 through a small angle when the lever 30 is displaced in the clockwise direction.

It is however understood that more or less than ten compartments 70 can be positioned on the base foil 60 and the cartridge base 55 can include as many peripheral cutouts as are necessary to ensure that each compartment is indexed to the required position by displacement of the lever 30. The base foil 60 is positioned axially aligned on top of the cartridge base 55.

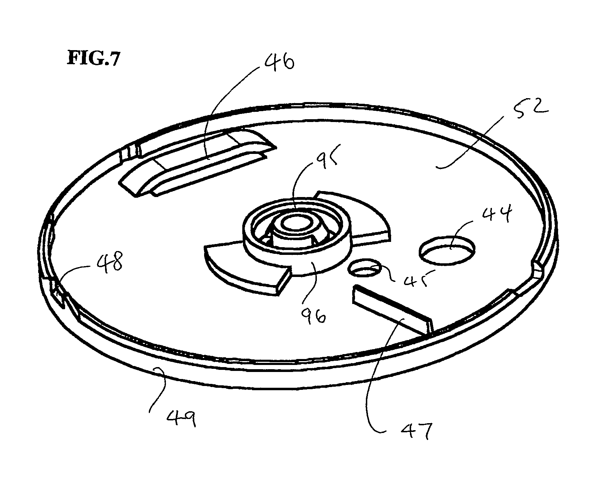

The lower cover 51 has an arcuate slot 9 through which the drive cam 18 extends to engage the cartridge base. The lower cover 51 of the cartridge 50 has a central upstanding circular spigot 84 that includes an internal upstanding ring 85. As shown in Figures 4 and 5 a pair of radially aligned wedges 87 and 86 extend outwardly and inwardly from the spigot 84 and inner wall of the lower cover 51. When the base foil compartments 70 have been filled and covered by the perforated layer 80 and sealed by the lidding foil 90, the laminated cartridge assembly is lowered into the lower cover 51 with the wedge shaped openers 87, 86 clearing the inner and outer slots 57, 58, 62, 63, 82 & 83 on the central apertures and outer peripheries of the base, base foil and perforated layers respectively. It should be noted that the lidding foil layer 90 does not have slots on the inner and outer peripheries which means that the foil rests on the wedges 86, 87. The cartridge 50 is completed by location of the upper cover 52 into locked engagement with the lower cover 51. As shown in Figure 7, the upper cover 52 has a peripheral downwardly extending skirt 49 with a rectangular cut-out 48 into which a lug 108 on the end of the drive cam 18 locates so movement of the drive cam 18 causes a rotational movement of the upper cover 52.

As shown in Figures 7 and 10, the upper cover 52 has a central tapered boss 95 that clips into the ring 85 in the lower cover 51. A downwardly extending annular flange 96 fits against the exterior of the ring 85 allowing the upper cover 52 to oscillate relative to the lower cover 51. The upper surface of the upper cover 52 includes a viewing aperture 44 and an air inlet aperture 45. A finger tab 46 extends down from the upper surface of the cover to provide ease of removal of the cartridge 50 from the inhaler body. The underside of the upper cover 52 also has an elongate downwardly extending bar 47 that, in use, engages the top of the lidding foil 90 to push back the lidding foil onto the perforated layer 80, base foil 60 and base 55 after the contents of a compartment have been ejected. The bar 47 thus partially reseals the compartment.

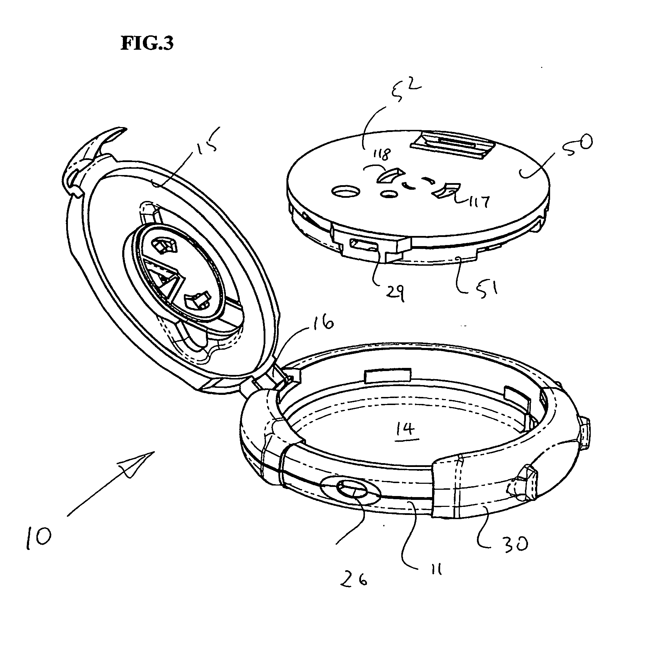

Figure 3 shows how the cartridge 50 can be lowered into the inhaler body 11. The cartridge 50 is gripped by the finger tab 46 and lowered into the recess 14 of the inhaler body 11 with outlet slot 29 aligned with the mouthpiece 26. The shape of the cartridge 50 is such that it can only be positioned in the inhaler in the correct position. The lever 30, through the drive cam (not shown), engages the cutouts 53 in the underside of the cartridge base 55 so that movement of the lever 30 has the effect of causing rotational movement of the disc base 55. The lever that drives the cartridge base 55 to rotate relative to the lower cover 51 of the cartridge 50 also has the effect of causing the upper cover 52 to oscillate on the lower cover 51 by contact between the lug 108 on the drive cam 18 and the cut-out 48 in the upper cover 52. The connection between the lever 30 and drive cam 18 introduces a small degree of free play or neutral movement.

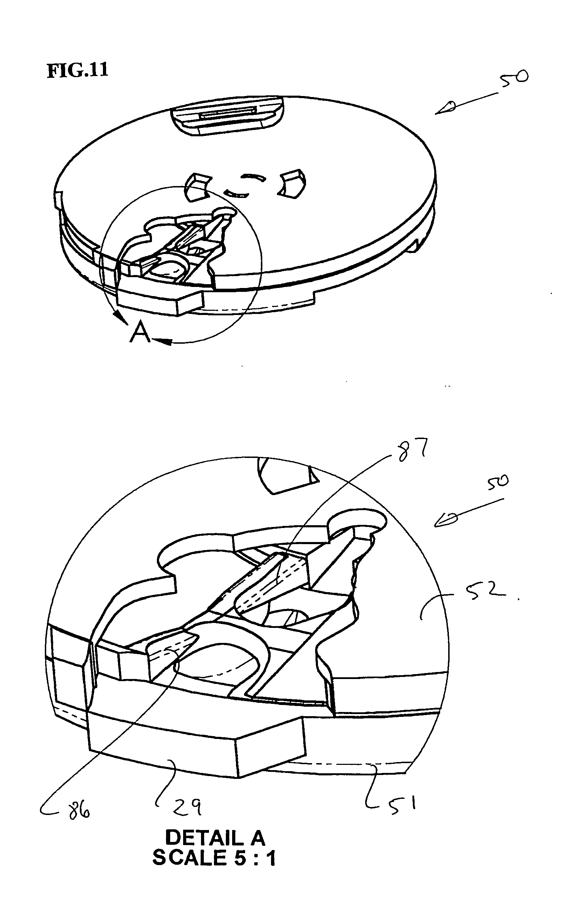

The role of the wedge shaped openers 86, 87 is illustrated with particular reference to Figures 10 and 11. As mentioned above, when the cartridge is assembled, the disc assembly sits in the lower cover 51 with the wedge shaped openers 86, 87 resting against the underside of the lidding foil layer 90. As the lever is actuated to cause the disc assembly to rotate relative to the lower cover 51, the inclined ramp on the upper surface of the openers 86, 87 has the effect of partially lifting the lidding foil 90 at the inner and outer sections from the top of the perforated layer 80 thereby exposing the powder within the compartment 70. The openers can either be positioned in a leading, central or trailing position in relation to the medicament compartment 70 on the assembly and as the disc continues to rotate, the openers lift the inner and outer sections of the lidding foil to expose the contents for removal upon inhalation and then allow the lidding foil to fall back into position against the perforated layer 80 thereby re-closing the compartment 70. The trailing bar 47 on the underside of the upper cover 52 then pushes the foil back against the base foil to partially reseal the compartment.

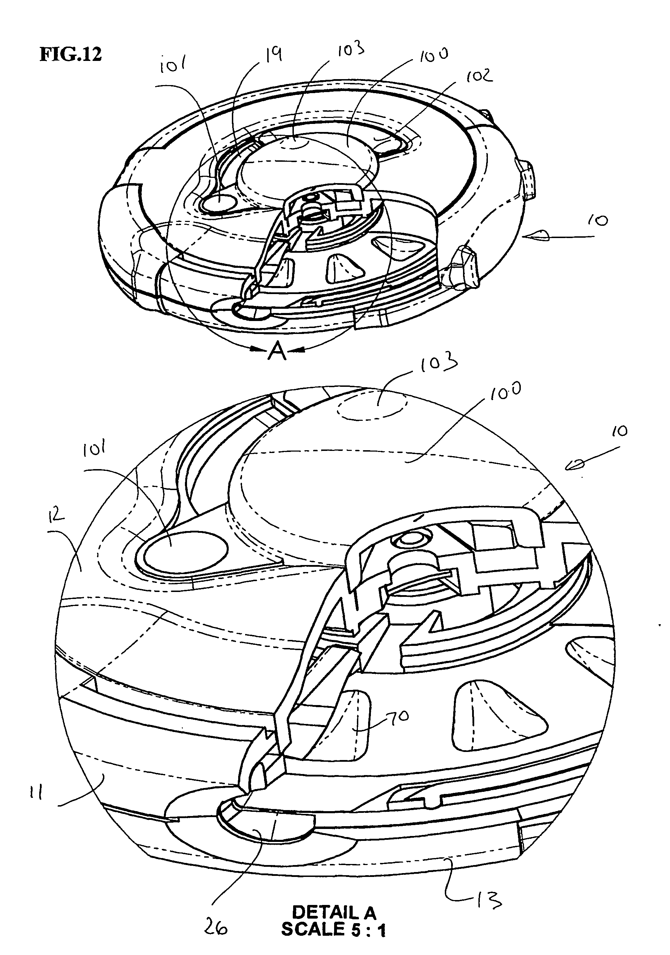

The lid 15 of the inhaler is shown in greater detail in Figures 2, 12 and 13. The lid has a circular shaped clear cover 100 with a viewing tab 101 on one side and an arcuate window 102 on the opposite side. The circular shaped clear cover 100 is obscured except for an area 103. There is a gap extending about 90° around the clear cover 100 defining the air inlet 19. The underside of the lid has a central spigot 104 which supports a flow indicator 110 and a flap valve 112. As air is drawn into the inhaler through the air inlet 19, the flexible flap valve 112 pivots open as shown in detail B. Any attempt to blow air back through the inhaler is prevented by the flap valve 112 moving to the closed position shown in detail A.

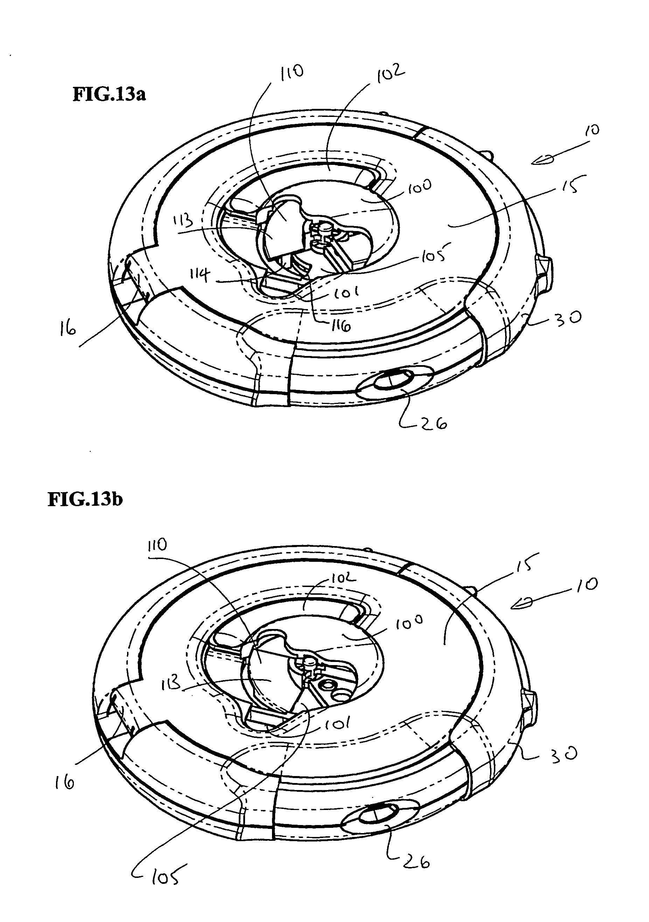

As shown in Figure 13, the flow indicator 110 is in the form of an arcuate flag 113 that has downward projections 114, that reside in grooves 116 in the underside of the lid. As air is drawn into the inhaler 10 it causes the flag 113 to move in the grooves 116 and to rise up a ramp to assume a visible position through the area 103 of the window 100. The ramp tends to hold the flag 113 in the operative position after inhalation. When the lever 30 is indexed to the closed position, the oscillating movement of the top of the cartridge 50 causes arcuate cut-outs 117 and 118 in the top 52 of the cartridge to engage the projections 114 to return the flag 113 to the inoperative and less visible position. The incoming air current is sufficient to drive the flag 113 to the operative position. Thus, as shown in Figure 13 the airflow is such that when the user inhales on the mouthpiece 26 air is drawn into the inhaler 10 via the air inlet 19 around the underside of the window 100 into the inhaler to move the flag 113 to the position shown in Figure 13b. At this stage with the flag 113 in the operative position the air flows in to the inhaler displacing the one way valve 112 and into the cartridge 50. The air flows through the air inlet 45 at the top of the cartridge and out under the lidding foil that has been prized upwardly by the openers 86, 87, through the perforated layer 80 across the top of the compartment 70 and out through the radially outer section of the compartment through the perforated layer 80 and the inhalation aperture 27 and mouth piece 26. The air current is such that it causes turbulence causing the powder to be drawn through the perforated layer 80 to be entrained in the air for expulsion. The perforated layer 80 has the role of preventing escape of powder without the air current so thus, if for some reason, the lidding foil 90 is removed from the compartment by accident the perforated layer 80 prevents escape of the powder and only allows powder escape when it is entrained in an air current. The perforations in the layer 80 also assist to control the particle size of the released medicament.

The lid also includes the small viewing tab 101 that exposes through magnification an arcuate line of numbering that would be positioned on the lidding foil 90 and exposed through the hole 44 in the upper cover 52. The numbering reflects the number of recesses 70 with unused doses so that the user of the inhaler can know how many doses remain in the cartridge.

The inhaler 10 also includes a number of other features that reduce inadvertent additional dosage and reduce the likelihood of accidental displacement of the medicament. It is only on a full displacement of the lever 30 to the right as shown in Figure 3 that opens the next dose and indexes the cartridge so the dose is positioned in line with the airflow passageway. The lever is connected to an arcuate band the drive cam 18 that locates on the inner surface of the body 11. The connection between the drive lever 30 and drive cam 18 introduces a small degree of free play or neutral movement. The lever is coupled to the drive cam having an aperture 109 so that only full displacement of the lever to the right as shown in Figure 4 moves the aperture 109 in the drive cam 18 into correct alignment with the aperture 27 of the mouthpiece 26 to open the air passageway. When the lever returns to the left or closed position the drive cam moves to close off the mouthpiece 26.

The lever 30 that closes off the airflow passageway and does not open this passageway until the lever has again been displaced fully to the right. As the lever 30 is displaced the openers 86, 87 lift the lidding foil 90 from the perforated layer 80 to expose the radially inner and outer sections of recessed compartment 70. By the time the lever 30 has moved to the fully displaced position the foil 90 has been lifted from the radially inner and outer sections of the compartment 70 to open the air passageway. At that time the air passageway is open to the mouthpiece 26 allowing inhalation. If the lever is closed i.e. returned to its original position to the left without taking the dose that dose will then be lost because it will be indexed into an inoperative position when the lever has moved again. Thus reducing the possibility of unintentional additional dosing.

The cover 52 that is positioned over the foils 60, 80 and 90 protects doses that are not used from escape into the inhaler so that once the cartridge is discarded any residual medicament is discarded with the cartridge. Because the openers 86, 87 only lifts the lidding foil 90 off the perforated layer 80 an unadministered dose becomes effectively sealed in its compartment 70 as it is indexed past the openers which allows the lidding foil to return to its former position with the bar 47 closing off the compartment 70.

The flap 112 operates as a one-way valve to ensure that exhalation does not have any effect on the medicament. The valve virtually prevents or at least minimises the amount of air that can be blown into the device so that exhalation does not dislodge or disturb a readied dose or for that matter disturb a dose that has not been administered. When in its uppermost position the one way valve 112 closes the air flow pathway exit to minimise the possibility of air flow over the unadministered dose.

The shape of the cutouts in the periphery of the base is such that when the last dose has been dispensed the lever cannot further rotate the disc so that the user becomes aware that the cartridge is empty and can thus replace the cartridge.

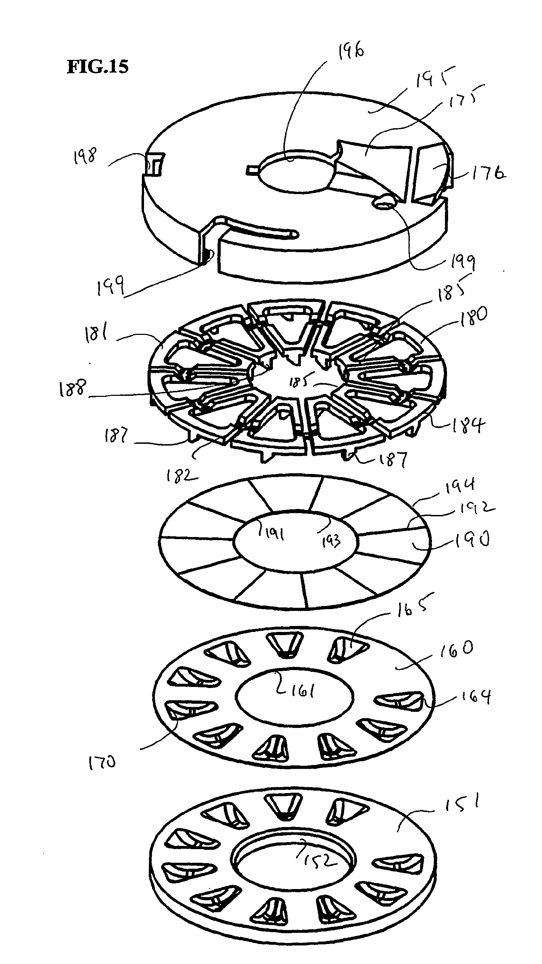

Figures 15 to 17 illustrate a second embodiment that utilises a different cartridge which is shown in Figure 15. The cartridge 150 has a slit top foil layer 190 provided with radial slits 192 that define segments that correspond to the position of each compartment. The top foil layer 190 has a central aperture 191 and is bonded to the lower foil 160 to seal off the compartments 170. A circular assembly 180 of flip top members 181 is bonded to the top foil 190. The assembly 180 comprises a plastics moulding in the form of a plurality of radially extending flip top members 181 that are interconnected by circumferentially extending webs 182. Each flip top member 181 comprises a radially outer arm 184 that is joined to a V-shaped inner arm 185 by the webs 182 that interconnect that flip top member 181 to the adjacent flip top members. The underside of both the radially outer and inner arms include downwardly projecting triangular shaped lugs 187 and 188. The bonding of the assembly 180 to the top foil 190 means that each segment includes a segmentally shaped piece of foil with the skeletal framework of the flip top members 181 transcribing the inner and outer circumferential edges 193 and 194 as well as the radial edges of the segment. Because the assembly 180 is bonded to the top foil 190, rotation of the disc base 151 causes rotation of the assembly 180, top foil 190 and lower foil 160 in unison relative to the cover 195.

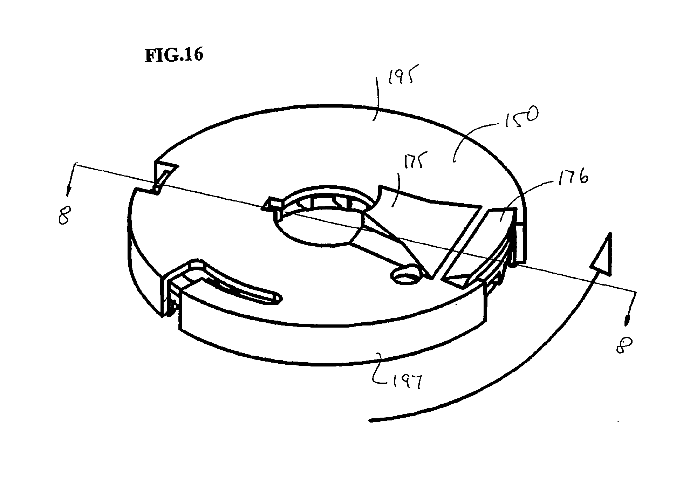

The assembly of the disc base 151, two foil layers 160 and 190 and flip top assembly 180 is then covered by a plastics cover 195 that has a central aperture 196 and a downwardly extending annular skirt 197 that covers the components. An arcuate cutout 198 is provided in the periphery of the skirt 197 of the cover 195 through which a lever (not shown), similar to the first embodiment, can extend to engage the disc base 151. The rotation of the disc base 151 and foil layers 160 and 190 and flip top assembly 180 relative to the cover 195 is illustrated in Figure 16. An arcuate cutout 199 is provided in the periphery of the skirt 197 of the cover 195 which, prevents the base rotating in the wrong direction by engaging the disc base 151.

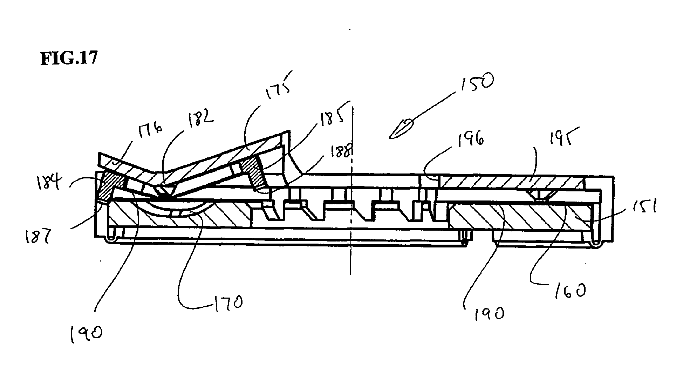

Displacement of the lever rotates the disc base 151 causing the inner and outer lugs 187, 188 on the flip top member 181 to ride up on radial projections on the base of the inhaler (not shown) to cause the arms 184, 185 of the flip top members 181 to flex upwardly as shown in Figure 17 about the central line or webs 182. Upward flexing of the flip top members 181 lifts the top foil 190 from the radially inner and outer edges of the compartments 170 causing an air passageway to form between the centre of the cartridge 150, the lifted inner flip top arm 184 the compartment 170 and the lifted outer flip top arm 185. In this way the airflow passageway is defined by the top foil 190, the flip top member 181 and the compartment 170. The cover 195 of the cartridge has inclined up standing portions 175 and 176 that are positioned to accommodate the flip top member 181 in the elevated position as shown in Figure 17. As the disc base 151 is further rotated the undersurface of the cover 195 forces the previously opened flip top members 181 down to the horizontal position, shown around the remainder of the periphery of the flip top assembly 180 in Figure 15. The radial slits 192 in the top foil 190 facilitate the upward movement of the flip top members 181 relative to the remainder of the foil 190.

The flip top assembly 180 has a dual role of displacing the top foil 190 layer from the lower foil 160 and thus exposing each compartment 170 whilst at the same time forming a framework for an air passageway that flows from the aperture in the top of the lid of the inhaler down through the centre of the inhaler and along the radial arms 184, 185 to pass through the end of the radial outer arm 185 and through the mouthpiece in the periphery of the body. The flip top members 181 lift the top foil 190 off the lower foil 160 and the radial edges of the compartment and the skeletal structure of the members 181 coupled with the foil surfaces provides the air passageway so that the user inhales through the mouthpiece drawing air down and into the inhaler and through the passageway. The air current picks up the powder in the exposed recess 170. The powder is then entrained in the air to leave the inhaler via the mouthpiece.

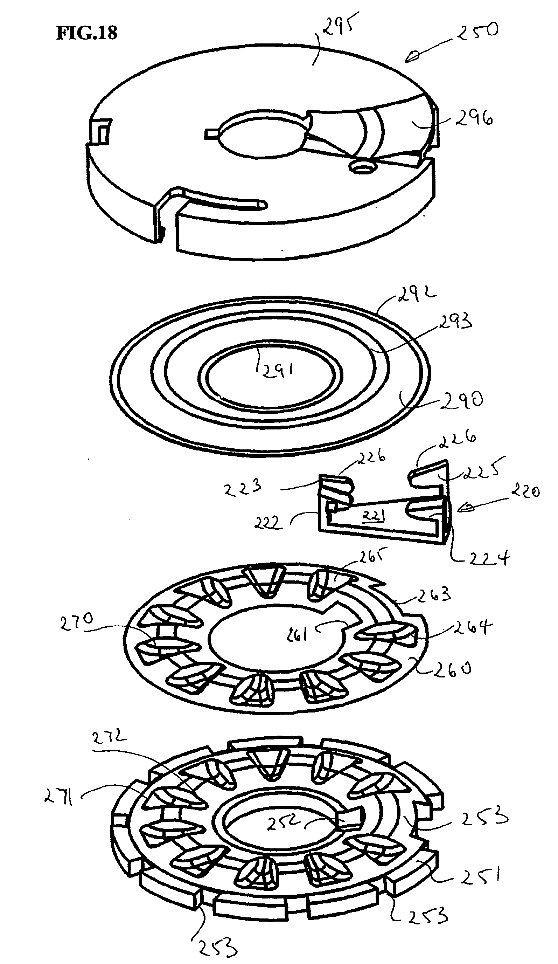

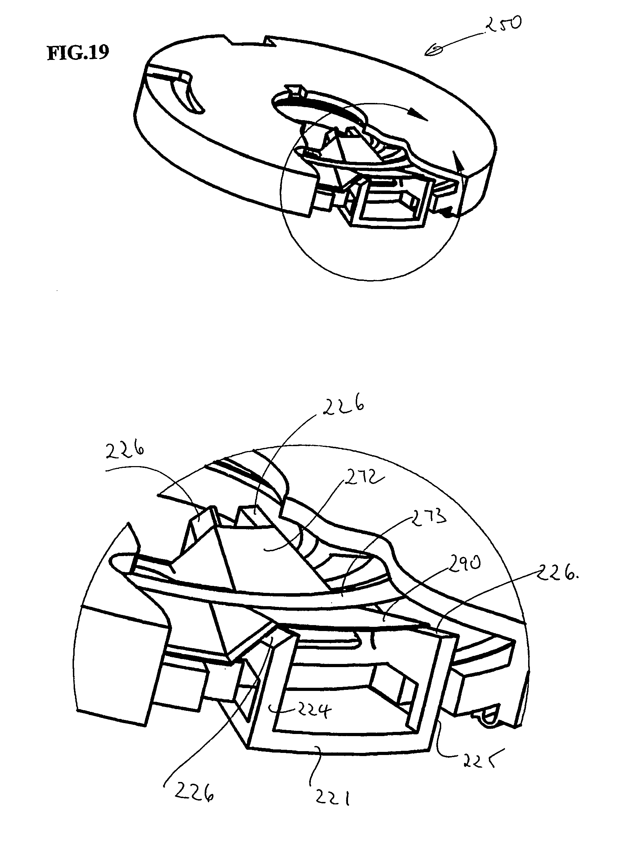

In the third embodiment shown in Figures 18 and 19, the disc base 251 and lower foil 260 are provided with radially inner and outer cutouts 252, 253, 262, 263 in the gap 268 between the first and last compartment 264, 265. The cut-outs 252, 253, 262, 263 accommodate a disc opener 220 in the form of a bracket having a flat base 221 terminating an upstanding posts 222, 223, 224, 225 at either end with the posts having inturned downwardly extending flanges 226. The posts and flanges 226 are positioned on the radially outer and radially inner end of the opener 220 and allow the opener to clip against the underside of the disc base 251 with the flat base 221 in parallel sliding contact with the underside of disc base 251 and the flanges 226 extending across the lower foil 260 surface but beneath the upper foil 290. The disc opener 220 is located in the cover 250 of the cartridge in a manner that it cannot rotate with the disc base 251 so that as the disc base is rotated the leading edges of the flanges 226 have the effect of lifting up the radially inner and outer edges of the top foil 290 on the adjacent compartment 270. As the disc base is indexed to the operative position as shown in Figure 19 the radially inner and radially outer edges of the top foil 290 are lifted clear of the compartment 270 and the air passageway is defined by the compartment base and the top foil 290 that has been raised at least adjacent the radially outer and radially inner edges of the compartment by the disc opening flanges 226.

To ensure that the top foil 290 lifts off a single compartment 270 the radially outer 271 and radially inner edges 272 of each compartment 270 are at a position lower than the centre of the compartment 273 so that the disc opener flanges 226 only have to lift the radially inner 291 and radial outer 292 edges of the top foil level with the centre 293. It is for this reason that the top foil 290 is illustrated with what appear to be concentric rings. The central ring 293 allows the radially inner and radially outer sections of the top foil 290 to lift into the open position. This arrangement provides a narrow passageway whereby the central portion of the top foil 290 remains above the recessed compartment 270 and the air current to ensure that the air current is in close proximity to the powdered medicament.

When the top foil 290 is bonded to the lower foil 260 there is no bond in the gap 268 between the first 264 and last 265 compartments (except for the compartment periphery - a sealing band surrounding the compartment) which means that it becomes a simple exercise to insert the disc opening flanges 226 between the foil surfaces in that gap 268 to complete assembly.

The cover 295 of the disc 250 is provided with a raised inclined section 296 over the position of the disc opener 220 to accommodate the upstanding posts and flanges 226.

### FEATURES OF THE PREFERRED EMBODIMENTS

The inhaler is reusable, whilst the empty cartridges are discarded.

Cartridges can be supplied with a range of dose number, medicament type and volume.

Full and partially full cartridges can be loaded into and removed from the inhaler as required - either well before a dose is required or just prior to use.

Loading of the cartridge does not open a dose for inhalation.

The dose is opened and prepared for inhalation by simply sliding the indexing lever.

The access to the mouthpiece is opened or closed by simply sliding the indexing lever.

The possibility of unintentional additional dosing is minimised.

Exhalation into the inhaler does not affect the effectiveness of the next dose from the cartridge.

The inhaler via the cartridge has a "doses remaining" indicator.

The inhaler has an indicator to indicate correct dosage received.

The cartridge covers and foils protect the user from residues in opened compartments of the cartridge.

Although in the preferred embodiments the inhaler comprises an inhaler body and disposable cartridge it is understood that in a simple form the inhaler may simply be like the cartridge that is without the external body. The cartridge would include a mechanism to displace the compartments and cause opening of each compartment when it is aligned with an outlet aperture that would serve as the mouthpiece.

### MEDICATIONS USED WITH THE INHALER

The inhaler may be used to provide medications selected from the following therapy areas: antiinfluenza, analgesic, anti-anginal preparation, antiallergic, anti-infective, anticancer, antihistamine, anti-inflammatory, antitussive, bronchodilator, cortiscosteroid, diuretic, anticholinergic, hormone, xanthine, osteoporosis, hypertension, therapeutic protein or peptide, vaccine, diagnostic agent or gene therapy agent.

The inhaler may be used to provide medications selected from the following group: zanamivir, codeine, dihydromorphine, ergotamine, fentanyl, morphine, diltiazem, cromoglycate, ketotifen, nedocromil, cephalosporins, penicillins, streptomycin, sulphonamides, tetracyclines, pentamidine, methapyrilene, beclomethasone dipropionate, fluticasone propionate, flunisolide, budesonide, rofleponide, mometsasone furoate, triamcinolone acetonide, noscapine, albuterol sulphate, salmeterol xinafoate, salmeterol, ephedrine, adrenaline, fenoterol, formoterol, isoprenaline, metaproterenol, phenylephrine, phenylpropanolamine, pirbuterol acetate, reproterol hydro chloride, rimiterol, terbutaline sulphate, isoetharine, tulobuterol, orciprenaline, adenosine 2a agonists, α4 integrin inhibitors, amiloride, ipratropium, tiotropium, atropine or oxitropium, cortisone, hydrocortisone or prednisolone, aminophylline, choline theophyllinate, lysine theophyllinate or theophylline, insulin or glucagon, or salts, esters, or solvates thereof, alone or in combination.

## Claims

1. An inhaler (10) for delivering metered doses of powdered medicament, the inhaler (10) having a plurality of compartments (70) spaced in an annular array and each containing a metered dose of the medicament, a lever (30) to displace the compartments (70) one by one into line with an inhalation aperture (27) that constitutes a mouthpiece (26), each compartment (70) including radially inner and outer edges, the plurality of compartments (70) being closed by a) sealing layer (90), **characterised in that** the inhaler (10) has a mechanism (86, 87) adapted to lift the inner and outer edges of the sealing layer (90) off the radially inner and outer edges of each compartment (70) one by one as it is indexed to align with the inhalation aperture (27) to open an air passageway defined by the compartment (70) and the sealing layer (90) so that, in use, on inhalation through the mouthpiece (26), air flow in the air flow passageway picks up and entrains the powder in the compartment (70) to be drawn with the air out of the inhaler (10) though the mouthpiece (26).

2. The inhaler (10) according to claim 1 wherein each compartment (70) is covered by a perforated layer (80) and the sealing layer (90) covers the perforated layer (80) with the lifting mechanism (86, 87) adapted to lift the inner and outer edges of the sealing layer (90) from the perforated layer (80).

3. The inhaler (10) according to claim 1 or claim 2 wherein the lever (30) is moveable from an inoperative position to an operative position in which one compartment (70) is indexed to align with the inhalation aperture (27) and the inner and outer edges of the sealing layer (90) are lifted off the compartment (70), the lever (30) being returnable to the in operative position.

4. The inhaler (10) according to claim 3 wherein the inhaler (10) has a sealing bar (47) that pushes down the inner and outer edges of the sealing layer (90) as the lever (30) is returned to the inoperative position.

5. The inhaler (10) according to any one of claims 1 to 4 wherein the inhaler (10) comprises a body (11) adapted to receive a cartridge (50) that carries the compartments (70), the inhalation aperture (27) being provided in the body (11) in a position aligned with an outlet aperture (29) in the cartridge (50), and the lever (30) being mounted on the body (11) to be displaceable from the inoperative position to the operative position in which a fresh compartment (70) is displaced and opened so that the opened compartment (70) is aligned with the inhalation aperture (27).

6. The inhaler according to claim 5 wherein the lever (30) includes a component that closes the inhalation aperture (27) in the inoperative position and opens the aperture (27) in the operative position.

7. The inhaler (10) according to claims 5 or 6 wherein the body (11) defines a recess (14) into which the cartridge (50) is located, a lid (15) being pivoted to the body (11) to close off the recess (14).

8. The inhaler (10) according to claim 7 wherein the lid (15) includes an air entry aperture (19).

9. The inhaler (10) according to claim 8 wherein the lid (15) includes indicator means (110) to provide a visual indication that air has been drawn through the inhaler (10).

10. The inhaler (10) according to claim 9 wherein the indicator means (110) comprises a member (113) displaceable in response to a minimum air flow.

11. The inhaler (10) according to claim 10 wherein the member (13) is displaceable from a start position to a finish position, the finish position reflecting minimum air flow.

12. The inhaler (10) according to any one of claims 5 to 11 wherein the cartridge (50) comprises a disc assembly mounted within upper and lower covers (52, 51), the disc assembly being axially rotatable relative to the lower cover (51).

13. The inhaler (10) according to claim 12 wherein the disc assembly comprises the array of spaced compartments (70) sealed by the sealing layer (90), which is disc shaped.

14. The inhaler (10) according to claim 13 wherein the array of compartments (70) are formed in a metal foil disc shaped sheet (60) that is positioned on a similarly formed disc shaped base member (55).

15. The inhaler (10) according to any one of claims 12 to 14 wherein the lifting mechanism comprises a pair of spaced projections (86, 87), and wherein the lower cover (51) has said pair of spaced projections (86, 87) that extend past slots (57, 58, 62, 63, 82, 83) in the disc assembly to engage the underside of the sealing layer (90) whereby rotation of the disc assembly past the projections (86, 87) causes the projections (86, 87) to lift the sealing layer (90) off the inner and outer edges of the compartments (70) one by one as the compartments (70) move over the projections (86, 87).

16. The inhaler (10) according to claims 12 to 15 when dependant on claim 11 wherein the upper cover (52) is capable of oscillating relative to the lower cover (51), the oscillation causing rotation of the member (113) to the start position.

17. The inhaler (10) according to any one of claims 1 to 11 wherein the lifting mechanism comprises a flip top member (181) secured to the sealing layer (190) and having portions (184, 185) adapted to flex upwardly relative to the compartment (170) to lift the sealing layers (190) off the inner and outer edges of the compartment (170).

18. The inhaler (10) according to claim 17 wherein the inhaler (10) includes means to flex the portions (184, 185) upwardly as the compartment (170) is displaced relative to the inhaler (10).

19. The inhaler (10) according to any one of claims 1 to 11 wherein the lifting mechanism comprises an opener in the form of wedge shaped members (86, 87) mounted spaced apart on a plate which is positioned beneath the array of compartments (70), the wedge shaped members (86, 87) engaging the underside of the sealing layer (90), the plate being fixed relative to the inhaler (10) so that displacement of the array of compartments (70) to relative to the inhaler (10) causes each compartment (70) to move past the wedge shaped members (86, 87) to lift the sealing layer (90) off the inner and outer edges of the compartment (70).

20. The inhaler (10) according to any one of the preceding claims wherein the inhaler (10) has a one way valve (112) that allows air to be drawn in through the inhaler (10) and out of the mouthpiece (26) but prevents flow of air in the reverse direction.

21. The inhaler (10) according to any one of the preceding claims wherein ten compartments (70) are spaced in an array.

## Patentansprüche

1. Ein Inhalator (10) für die Bereitstellung abgemessener Dosen eines pulverförmigen Medikaments, der Inhalator (10) hat dabei eine Vielzahl von Kammern (70), die in einer ringförmigen Anordnung voneinander getrennt sind und jeweils eine abgemessene Dosis des Medikaments enthalten, einen Hebel (30), um die Kammern (70), eine nach der anderen, in eine Linie mit einer Inhalationsöffnung (27) zu verstellen, die ein Mundstück (26) bildet, jede Kammer (70) schließt dabei strahlenförmig eine Innenkante und eine Außenkante ein, die Vielzahl der Kammern (70) ist durch eine Abdeckschicht (90) geschlossen, **dadurch gekennzeichnet, dass** der Inhalator (10) einen Mechanismus (86, 87) umfasst, der so angepasst ist, dass er die Innenkante und die Außenkante der Abdeckschicht (90) von der Innenkante und der Außenkante jeder Kammer (70), eine nach der anderen, abheben kann, damit er sich an der Inhalationsöffnung (27) ausrichten kann, um einen Luftdurchlass, der von der Kammer (70) und der Abdeckschicht (90) definiert wird, zu öffnen, so dass, bei Gebrauch, bei der Inhalation durch das Mundstück (26), der Luftstrom im Luftdurchlass Pulver aufnimmt und in die Kammer (70) mitreißt und das Pulver dann mit der Luft aus dem Inhalator (10) durch das Mundstück (26) gezogen wird.

2. Der Inhalator (10) gemäß Anspruch 1, wobei jede Kammer (70) von einer Perforationsschicht (80) abgedeckt ist und die Abdeckschicht die Perforationsschicht (80) per Hebemechanismus (86, 87) zudeckt, der so angepasst ist, dass er die Innenkante und die Außenkante der Abdeckschicht (90) von der Perforationsschicht (80) abhebt.

3. Der Inhalator (10) gemäß Anspruch 1 oder Anspruch 2, wobei der Hebel (30) von einer Ruheposition in eine Betriebsposition beweglich ist, in der sich eine Kammer (70) an der Inhalationsöffnung (27) ausrichtet und die Innenkante und Außenkante der Abdeckschicht (90) von der Kammer (70) abgehoben werden, der Hebel (30) kann dabei in die Ruheposition zurückgestellt werden.

4. Der Inhalator (10) gemäß Anspruch 3, wobei der Inhalator (10) eine Abdeckleiste (47) umfasst, die die Innenkante und Außenkante der Abdeckschicht (90) herunterdrückt, wenn der Hebel (30) in die Ruheposition zurückgestellt wird.

5. Der Inhalator (10) gemäß eines der Ansprüche 1 bis 4, wobei der Inhalator (10) ein Gehäuse (11) aufweist, das so angepasst ist, dass es eine Kassette (50) aufnehmen kann, die die Kammern (70) enthält, die Inhalationsöffnung (27) ist im Gehäuse (11) in einer Position untergebracht, die an der Auslassöffnung (29) in der Kassette (50) ausgerichtet ist und der Hebel (30) ist so am Gehäuse (11) montiert, dass er aus der Ruheposition in die Betriebsposition verstellbar ist, in der eine frische Kammer (70) eingestellt und geöffnet wird, so dass die geöffnete Kammer (70) an der Inhalationsöffnung (27) ausgerichtet werden kann.

6. Der Inhalator (10) gemäß Anspruch 5, wobei der Hebel (30) eine Komponente einschließt, die die Inhalationsöffnung (27) in der Ruheposition verschließt und die Öffnung (27) in der Betriebsposition öffnet.

7. Der Inhalator (10) gemäß Anspruch 5 oder 6, wobei das Gehäuse (11) eine Einbuchtung (14) definiert, in die die Kassette (50) eingesetzt wird, und wobei ein Deckel (15) auf das Gehäuse (11) geschwenkt wird, um die Einbuchtung (14) abzuschließen.

8. Der Inhalator (10) gemäß Anspruch 7, wobei der Deckel (15) eine Lufteinlassöffnung (19) einschließt.

9. Der Inhalator (10) gemäß Anspruch 8, wobei der Deckel (15) eine Anzeigevorrichtung (110) einschließt, um eine Sichtanzeige bereitzustellen, dass Luft durch den Inhalator (10) gesaugt wurde.

10. Der Inhalator (10) gemäß Anspruch 9, wobei die Anzeigevorrichtung (110) ein Teil (113) aufweist, das als Reaktion auf einen Mindestluftstrom verstellbar ist.

11. Der Inhalator (10) gemäß Anspruch 10, wobei das Teil (113) von einer Startposition zu einer Endposition verstellbar ist, die Endposition repräsentiert dabei einen Mindestluftstrom.

12. Der Inhalator (10) gemäß eines der Ansprüche 5 bis 11, wobei die Kassette (50) eine Scheibenanordnung aufweist, die innerhalb der oberen und unteren Abdeckung (52, 51) montiert ist, die Scheibenanordnung ist dabei axial im Verhältnis zur unteren Abdeckung (51) drehbar.

13. Der Inhalator (10) gemäß Anspruch 12, wobei die Scheibenanordnung die Anordnung der abgeteilten Kammern (70) aufweist, die von der scheibenförmigen Abdeckschicht (90) abgedichtet werden.

14. Der Inhalator (10) gemäß Anspruch 13, wobei die Anordnung der Kammern (70) aus einer scheibenförmigen Metallfolien-Platte (60) geformt ist, die auf einem ähnlich geformten scheibenförmigen Basisteil (55) aufliegt.

15. Der Inhalator (10) gemäß eines der Ansprüche 12 bis 14, wobei der Hebemechanismus ein Paar voneinander abgetrennter Vorsprünge (86, 87) aufweist, und wobei die untere Abdeckung (51) das besagte Paar voneinander abgetrennter Vorsprünge (86, 87) umfasst, die sich an Nuten (57, 58, 62, 63, 82, 83) vorbei in der Scheibenanordnung erstrecken, um die Unterseite der Abdeckschicht (90) einrasten zu lassen, wobei die Drehung der Scheibenanordnung vorbei an den Vorsprüngen (86, 87) dazu führt, dass die Vorsprünge (86, 87) die Abdeckschicht (90) von der Innenkante und Außenkante der Kammern (70), eine nach der anderen, abhebt, wenn sich die Kammern (70) über diese Vorsprünge (86, 87) hinwegbewegen.

16. Der Inhalator (10) gemäß Anspruch 12 bis 15, in Abhängigkeit von Anspruch 11, wobei sich die obere Abdeckung (52) im Verhältnis zur unteren Abdeckung (51) hin und her bewegen kann, die Schwankung verursacht dabei eine Drehung des Teils (113) zur Startposition.

17. Der Inhalator (10) gemäß eines der Ansprüche 1 bis 11, wobei der Hebemechanismus ein Klapp-Teil (181) aufweist, das an der Abdeckschicht (190) befestigt ist und Teile (184, 185) umfasst, die so angepasst sind, dass sie sich im Verhältnis zur Kammer (170) nach oben biegen, um die Abdeckschicht (190) von der Innenkante und der Außenkante der Kammer (170) abzuheben.

18. Der Inhalator (10) gemäß Anspruch 17, wobei der Inhalator (10) eine Vorrichtung einschließt, die die Teile (184, 185) nach oben biegt, wenn die Kammer (170) im Verhältnis zum Inhalator (10) verstellt wird.

19. Der Inhalator (10) gemäß eines der Ansprüche 1 bis 11, wobei der Hebemechanismus einen Öffner in Form von keilförmigen Teilen (86, 87) aufweist, die voneinander abgetrennt auf einer Platte montiert sind, die unter der Anordnung der Kammern (70) positioniert ist, die keilförmigen Teile (86, 87) lassen dabei die Unterseite der Abdeckschicht (90) einrasten, die Platte ist im Verhältnis zum Inhalator (10) befestigt, so dass die Verschiebung der Anordnung von Kammern (70) im Verhältnis zum Inhalator (10), dazu führt, dass sich jede Kammer (70) an den keilförmigen Teilen (86, 87) vorbeibewegt, um die Abdeckschicht (90) von der Innenkante und Außenkante der Kammer (70) abzuheben.

20. Der Inhalator (10) gemäß eines der vorhergehenden Ansprüche, wobei der Inhalator (10) ein Einwegventil (112) hat, das die Luft durch den Inhalator (10) einströmen und aus dem Mundstück (26) herausströmen lässt, aber einen Luftstrom in umgekehrter Richtung verhindert.

21. Der Inhalator (10) gemäß eines der vorhergehenden Ansprüche, wobei zehn Kammern (70) in einer Anordnung voneinander abgetrennt vorhanden sind.

## Revendications

1. Un inhalateur (10) destiné à l'administration de doses dosées d'un médicament en poudre, l'inhalateur (10) possédant une pluralité de compartiments (70) espacés selon un matrice annulaire et chacun d'eux contenant une dose dosée du médicament, un levier (30) destiné à déplacer les compartiments (70) un par un en ligne avec une ouverture d'inhalation (27) qui constitue une embouchure (26), chaque compartiment (70) comprenant des bords radialement intérieur et extérieur, la pluralité de compartiments (70) étant fermés par une couche d'étanchéité (90), **caractérisé en ce que** l'inhalateur (10) possède un mécanisme (86, 87) adapté de façon à soulever les bords intérieur et extérieur de la couche d'étanchéité (90) des bords radialement intérieur et extérieur de chaque compartiment (70) un par un dans la mesure où il indexé de façon à aligner l'ouverture d'inhalation (27) de façon à ouvrir un conduit d'air défini par le compartiment (70) et la couche d'étanchéité (90) de sorte que, en utilisation, lors d'une inhalation par l'intermédiaire de l'embouchure (26), un flux d'air dans le conduit de flux d'air prélève et entraîne la poudre dans le compartiment (70) destinée à être aspirée avec l'air en dehors de l'inhalateur (10) au travers de l'embouchure (26).

2. L'inhalateur (10) selon la Revendication 1 où chaque compartiment (70) est recouvert par une couche perforée (80) et la couche d'étanchéité (90) recouvre la couche perforée (80) avec le mécanisme de levage (86, 87) adapté de façon à soulever les bords intérieur et extérieur de la couche d'étanchéité (90) de la couche perforée (80).

3. L'inhalateur (10) selon la Revendication 1 ou 2 où le levier (30) est déplaçable d'une position non opérationnelle à une position opérationnelle dans laquelle un compartiment (70) est indexé de façon à s'aligner avec l'ouverture d'inhalation (27) et les bords intérieur et extérieur de la couche d'étanchéité (90) sont soulevés du compartiment (70), le levier (30) pouvant être retourné vers la position opérationnelle.

4. L'inhalateur (10) selon la Revendication 3 où l'inhalateur (10) possède une barre d'étanchéité (47) qui pousse vers le bas les bords intérieur et extérieur de la couche d'étanchéité (90) à mesure que le levier (30) est retourné vers la position non opérationnelle.

5. L'inhalateur (10) selon l'une quelconque des Revendications 1 à 4 où l'inhalateur (10) comprend un corps (11) adapté de façon à recevoir une cartouche (50) qui comporte les compartiments (70), l'ouverture d'inhalation (27) étant placée dans le corps (11) dans une position alignée avec une ouverture de sortie (29) dans la cartouche (50) et le levier (30) étant monté sur le corps (11) de façon à être déplaçable de la position non opérationnelle vers la position opérationnelle dans laquelle un nouveau compartiment (70) est déplacé et ouvert de sorte que le compartiment ouvert (70) soit aligné avec l'ouverture d'inhalation (27).

6. L'inhalateur (10) selon la Revendication 5 où le levier (30) comprend un composant qui ferme l'ouverture d'inhalation (27) dans la position non opérationnelle et ouvre l'ouverture (27) dans la position opérationnelle.

7. L'inhalateur (10) selon les Revendications 5 ou 6 où le corps (11) définit un évidement (14) dans lequel se situe la cartouche (50), un couvercle (15) étant pivoté vers le corps (11) de façon à refermer l'évidement (14).

8. L'inhalateur (10) selon la Revendication 7 où le couvercle (15) comprend une ouverture d'entrée d'air (19).

9. L'inhalateur (10) selon la Revendication 8 où le couvercle (15) comprend un moyen d'indication (110) destiné à fournir une indication visuelle indiquant que de l'air a été aspiré au travers de l'inhalateur (10).

10. L'inhalateur (10) selon la Revendication 9 où le moyen d'indication (110) comprend un élément (113) déplaçable en réponse à un flux d'air minimal.

11. L'inhalateur (10) selon la Revendication 10 où l'élément (113) est déplaçable d'une position de départ vers une position d'arrivée, la position d'arrivée indiquant un flux d'air minimal.

12. L'inhalateur (10) selon l'une quelconque des Revendications 5 à 11 où la cartouche (50) comprend un ensemble disque monté à l'intérieur d'éléments de recouvrement supérieur et inférieur (52, 51), l'ensemble disque étant axialement pivotable par rapport à l'élément de recouvrement inférieur (51).

13. L'inhalateur (10) selon la Revendication 12 où l'ensemble disque comprend la matrice de compartiments espacés (70) scellée par la couche d'étanchéité (90) qui est en forme de disque.

14. L'inhalateur (10) selon la Revendication 13 où la matrice de compartiments (70) est formée dans une feuille de métal en forme de disque (60) qui est positionnée sur un élément base en forme de disque formé de manière similaire (55).

15. L'inhalateur (10) selon l'une quelconque des Revendications 12 à 14 où le mécanisme de levage comprend une paire de saillies espacées (86, 87), et où l'élément de recouvrement inférieur (51) possède ladite paire de saillies espacées (86, 87) qui s'étendent devant des fentes (57, 58, 62, 63, 82, 83) dans l'ensemble disque de façon à entrer en prise avec le côté inférieur de la couche d'étanchéité (90), grâce à quoi une rotation de l'ensemble disque devant les saillies (86, 87) amène les saillies (86, 87) à soulever la couche d'étanchéité (90) des bords intérieur et extérieur des compartiments (70) un par un à mesure que les compartiments (70) se déplacent au-dessus des saillies (86, 87).

16. L'inhalateur (10) selon les Revendications 12 à 15 lorsqu'elles dépendent de la Revendication 11 où l'élément de recouvrement supérieur (52) est capable d'osciller par rapport à l'élément de recouvrement inférieur (51), l'oscillation entraînant la rotation de l'élément (113) vers la position de départ.

17. L'inhalateur (10) selon l'une quelconque des Revendications 1 à 11 où le mécanisme de levage comprend un élément à abattant (181) fixé à la couche d'étanchéité (190) et possédant des parties (184, 185) adaptées de façon à fléchir vers le haut par rapport au compartiment (170) de façon à soulever la couche d'étanchéité (190) des bords intérieur et extérieur du compartiment (170).

18. L'inhalateur (10) selon la Revendication 17 où l'inhalateur (10) comprend un moyen de flexion des parties (184, 185) vers le haut à mesure que le compartiment (170) est déplacé par rapport à l'inhalateur (10).

19. L'inhalateur (10) selon l'une quelconque des Revendications 1 à 11 où le mécanisme de levage comprend un dispositif d'ouverture de la forme d'éléments cunéiformes (86, 87) montés espacés sur une plaque qui est positionnée sous la matrice de compartiments (70), les éléments cunéiformes (86, 87) entrant en prise avec le côté inférieur de la couche d'étanchéité (90), la plaque étant fixe par rapport à l'inhalateur (10) de sorte qu'un déplacement de la matrice de compartiments (70) par rapport à l'inhalateur (10) amène chaque compartiment (70) à se déplacer devant les éléments cunéiformes (86, 87) de façon à soulever la couche d'étanchéité (90) des bords intérieur et extérieur du compartiment (70).

20. L'inhalateur (10) selon l'une quelconque des Revendications précédentes où l'inhalateur (10) possède une soupape antireflux (112) qui permet à de l'air d'être aspiré au travers de l'inhalateur (10) et en dehors de l'embouchure (26) mais empêche un flux d'air dans la direction inverse.

21. L'inhalateur (10) selon l'une quelconque des Revendications précédentes où dix compartiments (70) sont espacés dans une matrice.
